# EUROPEAN PATENT SPECIFICATION

(11) **EP 4 481 512 B1**
(45) Date of publication and mention of the grant of the patent: **30.09.2026**
(21) Application number: 23755826.7
(22) Date of filing: 16.02.2023
(51) Int. Cl.: G04G 17/00, G04G 21/00, H01H 13/14, G04G 17/02, G04G 17/08, G04G 21/02, G04G 3/00, G04C 3/00

(54) **CONTROL COMPONENT AND WEARABLE DEVICE**
STEUERUNGSKOMPONENTE UND WEARABLE-VORRICHTUNG
ÉLÉMENT CONSTITUTIF DE COMMANDE ET DISPOSITIF POUVANT ÊTRE PORTÉ SUR SOI

(30) Priority: 18.02.2022 CN 202220343114 U
(43) Date of publication of application: 25.12.2024
(73) Proprietor: Huawei Technologies Co., Ltd., Shenzhen, Guangdong 518129 (CN)
(72) Inventor: LIU, Xiaogang, Shenzhen, Guangdong 518129 (CN); YANG, Wenjian, Shenzhen, Guangdong 518129 (CN); FU, Shifeng, Shenzhen, Guangdong 518129 (CN); REN, Yanming, Shenzhen, Guangdong 518129 (CN); WANG, Gang, Shenzhen, Guangdong 518129 (CN)
(74) Representative: Gill Jennings & Every LLP
(86) International application number: PCT/CN2023/076353
(87) International publication number: WO 2023/155824

(56) References cited:
- EP-A1- 3 451 117
- CN-A- 109 144 173
- CN-A- 111 065 984
- CN-U- 210 403 562
- CN-U- 212 342 511
- CN-U- 212 342 511
- CN-U- 214 125 763
- CN-U- 214 125 763
- CN-U- 214 378 142
- CN-U- 217 954 924
- JP-A- 2007 115 630

## Description

### BACKGROUND

Development of science and technology encourages people with increasingly high requirements for intelligent detection of health metrics in scenarios such as exercise, sleep, work, and leisure, so that functions such as heart rate detection, blood oxygen saturation measurement, and body temperature measurement emerge for intelligent wearable products. This is more demanding for structure designs and material selection of wearable products.

Wearable devices such as a smartwatch can detect heart rates for users by using an electrocardiograph (electrocardiograph, ECG) structure. Generally, electrodes of the ECG structure include a crown cap of the smartwatch and a smartwatch rear surface (away from a display) in contact with a wrist of the user. The user presses the crown cap of the smartwatch, to control an ECG function of the smartwatch by using a crown stem (or an operating stem) connected to the crown cap (or an operating cap). When the user presses the crown cap, the crown stem slides relative to a housing of the smartwatch, in other words, there is a gap between the crown stem and the housing of the smartwatch. For insulation between the crown stem and the housing of the smartwatch, an insulation plastic part is generally disposed therebetween. However, aging of the insulation plastic part and water leakage into the smartwatch result in conductivity between the crown stem and the housing of the smartwatch. That is why the insulation plastic part has poor insulation effect. EP 3 451 117 A1 discloses a control mechanism as defined in the preamble of claim 1.

### SUMMARY

The present invention provides a control mechanism and a wearable device in accordance with claims 1 and 6. Preferred embodiments are defined in claims 2-5. The control mechanism can not only avoid a poor insulation effect that may be caused by aging of or water leakage into a plastic part when the plastic part is used for insulation, but also simplify a structure of a wearable device like a smartwatch, thereby reducing production costs of the smartwatch.

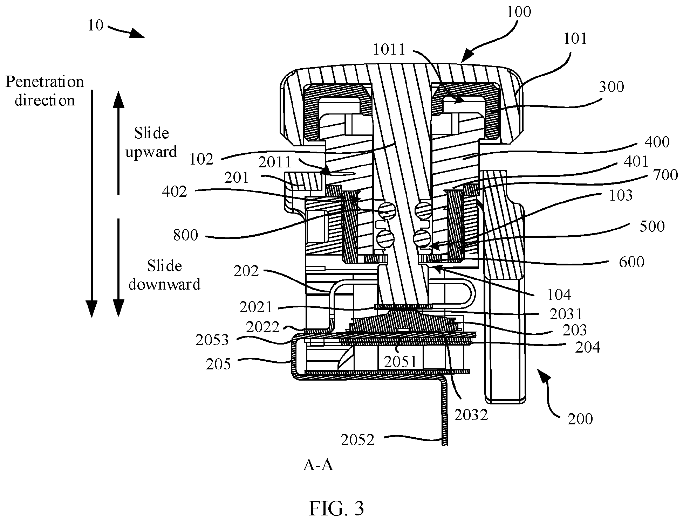

According to a first aspect, there is provided a control mechanism, where the control mechanism includes a fastener and an operating part isolated from the fastener through insulation. The fastener is provided with a through hole, the operating part penetrates the through hole, and an insulation layer is coated on at least a segment of an inner circumferential surface that is of the through hole and that is opposite to the operating part and/or on an outer circumferential surface that is of the operating part and that is opposite to the segment of the inner circumferential surface; and/or an insulation layer is coated on a contact surface on which the operating part abuts against an outer edge of the through hole in a penetration direction and/or on a surface that is of the outer edge of the through hole and that is opposite to the contact surface; and the fastener and the operating part are isolated through insulation by using the insulation layer. The penetration direction is a direction perpendicular to a plane on which an end face of the through hole is located, for example, a direction shown in FIG. 3 in which the operating cap points to the operating stem. In some implementations, the penetration direction may be an axial direction of the operating stem of the operating part below.

To be specific, the insulation layer may be coated on the inner circumferential surface that is of the through hole and that is opposite to the operating part, or may be coated on the outer circumferential surface that is of the operating part and that is opposite to the segment of the inner circumferential surface. Alternatively, the insulation layer is coated on both the inner circumferential surface that is of the through hole and that is opposite to the operating part and the outer circumferential surface opposite to the segment of the inner circumferential surface.

Alternatively, in a possible implementation, if the outer edge of the through hole of the operating part abuts against the operating part in the penetration direction, the insulation layer is coated on the outer edge of the through hole that abuts against the operating part, or the insulation layer is coated on the contact surface on which the operating part abuts against the through hole, or the insulation layer is coated on both the outer edge of the through hole that abuts against the operating part and the contact surface on which the operating part abuts against the through hole.

Then, a manner in which a plastic part is used for insulation in an original insulation solution is replaced with this manner, so as to resolve a problem of a poor insulation effect of the plastic part due to aging of or water leakage into the plastic part. In addition, a manner in which an insulation coating is used to replace the plastic part can also simplify a structure of a smartwatch, reduce processing difficulty, reduce a quantity of components, improve assembly efficiency, and reduce production costs of the smartwatch.

With reference to the first aspect, in a possible implementation, a thickness of the insulation layer ranges from 2 microns to 5 microns. To be specific, the thickness of the insulation layer is limited in a range. For example, the thickness of the insulation layer may be 3 microns. The thickness is a size of the insulation layer in a direction perpendicular to the outer circumferential surface or the contact surface.

With reference to the first aspect, in a possible implementation, the insulation layer includes diamond-like carbon (diamond-like carbon, DLC). It may be understood that in another possible implementation, the insulation layer may alternatively include another material.

With reference to the first aspect, the operating part is shaped like T, the operating part includes a horizontal operating cap and a vertical operating stem, the operating stem penetrates the through hole and slides relative to the through hole in the penetration direction, and the outer circumferential surface is an outer circumferential surface of the operating stem. To be specific, the operating stem is formed by the operating cap and the operating stem. The operating stem extends along a center of the operating cap in the penetration direction, penetrates the through hole, and may slide up and down relative to the through hole in the penetration direction. In this case, the operating stem is in contact with the through hole on the fastener. Therefore, to achieve an insulation effect between the fastener and the operating stem, the insulation layer needs to be coated on the outer circumferential surface that is of the operating stem and that is opposite to the through hole. That is, in this case, the outer circumferential surface is the outer circumferential surface of the operating stem.

With reference to the first aspect, in a possible implementation, the operating part is shaped like T, the operating part includes a horizontal operating cap, a vertical operating stem, and a crown tube sleeved on the operating stem, the operating stem is slidable relative to the crown tube in the penetration direction, the crown tube penetrates the through hole, the crown tube is unmovable relative to the through hole, and the outer circumferential surface is an outer circumferential surface of the crown tube. It may be understood that, a size of the smartwatch is small, and the through hole disposed on a housing of the smartwatch is also small. Therefore, the operating stem is thin and easy to bend. To protect the operating stem, a crown tube is generally sleeved on an outer circumference of the operating stem, to protect the operating stem. In this case, because the crown tube is in contact with the through hole, to achieve an insulation effect between the fastener and the crown tube, an outer circumferential surface that is of the crown tube and that is opposite to the through hole needs to be coated with the insulation layer.

With reference to the first aspect, in a possible implementation, the operating part is shaped like T, the operating part includes a horizontal operating cap, a vertical operating stem, and a crown tube sleeved on the operating stem, the operating stem is slidable relative to the crown tube in the penetration direction, the crown tube penetrates the through hole, the crown tube is unmovable relative to the through hole, and the contact surface is a surface on which the crown tube abuts against the outer edge of the through hole in the penetration direction. To be specific, when the crown tube abuts against the outer edge of the through hole in the penetration direction, to achieve an insulation effect between the fastener and the crown tube, the surface on which the crown tube abuts against the outer edge of the through hole needs to be coated with the insulation layer, or the outer edge that is of the through hole and that abuts against the crown tube needs to be coated with the insulation layer.

With reference to the first aspect, the fastener includes a housing, the through hole is provided on an outer circumferential surface of the housing, a metal dome switch is disposed inside the housing, an end that is of the operating stem and that is away from the operating cap elastically abuts against a top of the metal dome switch, the operating stem slidably penetrates the through hole in the penetration direction, and the metal dome switch is switched on/off by pressing/releasing the operating cap. To be specific, the fastener may be a housing structure, for example, a housing of the smartwatch. The through hole is provided on an outer circumferential surface of the housing, the metal dome switch is disposed inside the housing, an end of the operating stem elastically abuts against the top of the metal dome switch, and the operating stem can slide relative to the through hole in the penetration direction. In this way, the operating stem can be controlled to slide up and down by pressing/releasing the operating cap, so as to control switch-on/off of the metal dome switch.

In addition, in a possible implementation, the housing may be of a circle shape or a square shape, and if the housing is of a circle shape, the through hole is provided in a circumferential direction of the housing.

With reference to the first aspect, an elastomer and a first bracket supporting the metal dome switch are further disposed inside the housing, one end of the elastomer is fastened to the first bracket, the other end of the elastomer abuts against the top of the metal dome switch, and the end that is of the operating stem and that is away from the operating cap elastically abuts against the metal dome switch through the other end of the elastomer. To be specific, one end of the elastomer is disposed between an end that is of the operating stem and that abuts against the top of the metal dome switch and the top of the metal dome switch. In this way, when the operating cap is pressed, the operating stem may be used to press the elastomer and the top of the metal dome switch, for switch-on of the metal dome switch. It may be understood that in this case, when the operating stem is pressed, the elastomer deforms downward, and generates an upward resilience force. Then, after the operating cap is released, the operating stem drives the operating cap to slide upward under the resilience force of the elastomer, for restoration of the operating cap to an original position.

In addition, in a possible implementation, the other end of the elastomer may be in clearance fit with the top of the metal dome switch, or may be fastened to the top of the metal dome switch. It may be understood that, when the other end of the elastomer is in clearance fit with the top of the metal dome switch, the other end of the elastomer and the top of the metal dome switch may move synchronously. To be specific, when the operating cap is pressed and the operating stem slides downward, the other end of the elastomer and the top of the metal dome switch may move downward synchronously, so that the metal dome switch is switched on more timely.

Because the elastomer requires high costs, to further reduce costs of the smartwatch, an end of the operating stem may elastically abut against the top of the metal dome switch through a spring.

With reference to the first aspect, in a comparative implementation, the operating part further includes a sleeve structure disposed between the crown tube and the operating stem, the sleeve structure includes a body part and an extension part, the body part penetrates the crown tube, the extension part abuts against the crown tube in the penetration direction, and the operating stem is slidable relative to the sleeve structure in the penetration direction. The purpose is to enable the operating stem to slide up and down relative to the sleeve structure.

With reference to the first aspect, in a comparative implementation, the fastener includes a housing, the through hole is provided on an outer circumferential surface of the housing, and a metal dome switch and a first elastic structure are disposed inside the housing. The crown tube, and the body part and the extension part of the sleeve structure enclose an accommodating cavity, the accommodating cavity accommodates the first elastic structure, one end of the first elastic structure in the penetration direction abuts against the extension part of the sleeve structure, and the other end is disposed opposite to the operating cap. An end that is of the operating stem and that is away from the operating cap abuts against a top of the metal dome switch, and the metal dome switch is switched on/off by pressing/releasing the operating cap.

To be specific, the crown tube, and the body part and the extension part of the sleeve structure enclose the accommodating cavity, the first elastic structure is disposed in the accommodating cavity, one end of the first elastic structure in the penetration direction abuts against the extension part of the sleeve structure, the other end of the first elastic structure is disposed opposite to the operating cap, and the end that is of the operating stem and that is away from the operating cap abuts against the top of the metal dome switch, so that the end that is of the operating stem and that is away from the operating cap elastically abuts against the top of the metal dome switch. It may be understood that when the operating cap is pressed, the operating cap abuts against the other end of the first elastic structure, so that the first elastic structure deforms downward, and generates an upward resilience force. At the same time, the operating stem is driven by the operating cap to press the top of the metal dome switch downward, for switch-on of the metal dome switch. When the operating cap is released, the operating cap drives, under the resilience force of the first elastic structure, the operating stem to slide upward relative to the sleeve structure, for switch-off of the metal dome switch. In this way, the metal dome switch can be switched on and off. In addition, in a possible implementation, the other end of the first elastic structure may be in clearance fit with the crown cap.

With reference to the first aspect, in a comparative implementation, the first elastic structure is a spring, and the spring is sleeved on an outer circumferential surface of the body part.

According to a second aspect, there is further provided a wearable device. The wearable device includes the control mechanism in any one of the possible implementations of the first aspect and a display, and the display is disposed on a fastener of the control mechanism. In other words, in a possible implementation, the wearable device may be a device with a display, for example, a smartwatch.

### BRIEF DESCRIPTION OF DRAWINGS

To describe the technical solutions in embodiments of the present invention more clearly, the following briefly describes the accompanying drawings for describing embodiments. It is clear that the accompanying drawings in the following description show merely some embodiments the present invention, and a person of ordinary skill in the art may still derive other drawings from these accompanying drawings without creative efforts.
FIG. 1(A) is a diagram of a structure of an example of a smartwatch 1 according to some embodiments;
FIG. 1(B) is an exploded view of a partial structure of the smartwatch 1 in FIG. 1(A);
FIG. 2(A) is a top view of a partial structure in FIG. 1(A);
FIG. 2(B) is a side view of FIG. 2(A);
FIG. 3 is a sectional view of an operating part 100 and a fastener 200 in an S2 area of FIG. 2(A) along an A-A section in FIG. 2(A);
FIG. 4 is a sectional view of an operating part 100 and a fastener 200 in an S1 area of FIG. 2(B) along a B-B section in FIG. 2(B);
FIG. 5(A) is a diagram of a structure of an example of a smartwatch 1 according to some embodiments;
FIG. 5(B) is a diagram of a structure of an example of a smartwatch 1 according to some embodiments;
FIG. 6 is a diagram of a control structure 10 in an example of a smartwatch according to some embodiments; and
FIG. 7 is a diagram of a hardware structure of an example of a wearable device according to some embodiments.

### Reference numerals:

10-control mechanism;
100-operating part;
101-operating cap;
1011-first annular groove;
102-operating stem;
103-first slot;
104-second slot;
105-fourth slot;
106-fifth slot;
200-fastener;
201-housing;
2011-through hole;
202-elastomer;
2021-first end part;
2022-second end part;
203-metal dome switch;
203 1-top;
2032-bottom;
204-FPC board;
205-first bracket;
2051-first portion;
2052-second portion;
207-third bracket;
208-first fastening structure;
209-second fastening structure;
300-second bracket;
400-crown tube;
401-flange part;
4011-first stepped surface;
4012-second stepped surface;
402-third slot;
500-first annular structure;
600-circlip;
700-second waterproof part;
800-first waterproof part;
900-first elastic structure;
901-first end;
902-second end;
1000-first sleeve structure;
1001-flange part;
1002-body part;
1003-extension part;
1100-third waterproof part; and
1200-first insulation part.

### DESCRIPTION OF EMBODIMENTS

The following describes embodiments of the present invention with reference to the accompanying drawings in embodiments of the present invention. Terms used in implementations of embodiments of the present invention are merely used to explain specific embodiments.

The following uses an example in which a wearable device is a smartwatch 1 for description. It may be understood that the wearable device in embodiments of the present invention may alternatively be another wearable device, for example, smart glasses, a smart headset, or the like.

As described in the background, currently, a crown stem of the smartwatch 1 is insulated from a watch case or a housing by adding a plastic part, for example, a gap formed between the crown stem of the smartwatch 1 and the housing is provided with an insulation part that is made of plastic or rubber and that fits the gap. A pair of opposite surfaces in the insulation part respectively press against an outer circumferential surface of the close crown stem and an inner hole surface of a through hole on the housing, for insulation between the crown stem and the housing. However, in one aspect, the insulation part made of plastic or rubber is prone to aging and loosening, and in another aspect, when water enters the gap, insulation cannot be implemented by using the insulation part made of plastic or rubber.

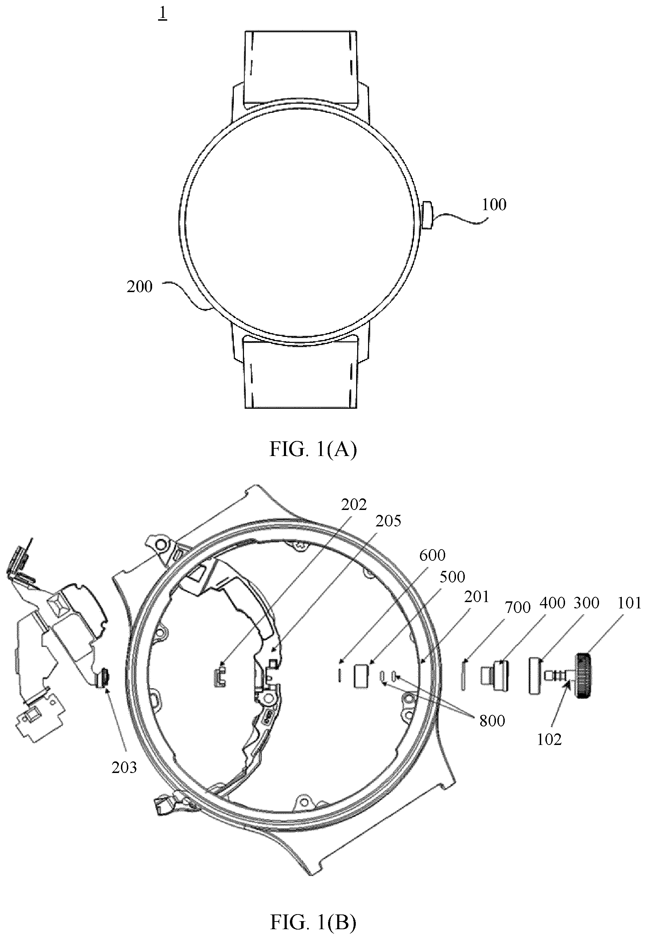

Therefore, to resolve the foregoing technical problem and simplify a structure of the smartwatch 1, this application provides a control mechanism 10. The following describes the control mechanism 10 with reference to FIG. 1 to FIG. 4. FIG. 1(A) shows an overall structure of the smartwatch 1, and FIG. 1(B) is an exploded view of a structure of the smartwatch 1 in FIG. 1.

As shown in FIG. 1(A), the control mechanism 10 includes a fastener 200 and an operating part 100 isolated from the fastener 200 through insulation. The fastener 200 is provided with a through hole, the operating part 100 penetrates the through hole 200, and an insulation layer is coated on at least a segment of an inner circumferential surface that is of the through hole and that is opposite to the operating part 100 and/or on an outer circumferential surface that is of the operating part 100 and that is opposite to the segment of the inner circumferential surface; and/or an insulation layer is coated on a contact surface on which the operating part 100 abuts against an outer edge of the through hole in a penetration direction and/or on a surface that is of the outer edge of the through hole and that is opposite to the contact surface; and the fastener 200 and the operating part 100 are isolated through insulation by using the insulation layer. The control mechanism 10 can not only avoid a poor insulation effect that may be caused by aging of or water leakage into a plastic part when the plastic part is used for insulation, but also simplify the structure the smartwatch 1, thereby reducing production costs of the smartwatch 1.

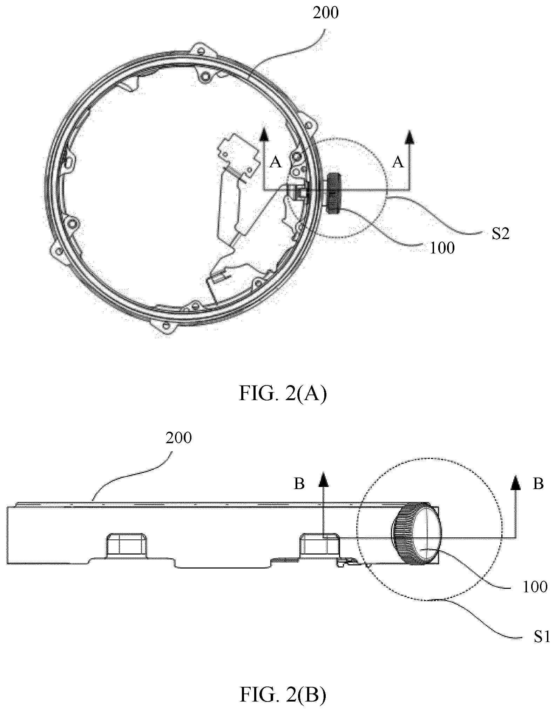

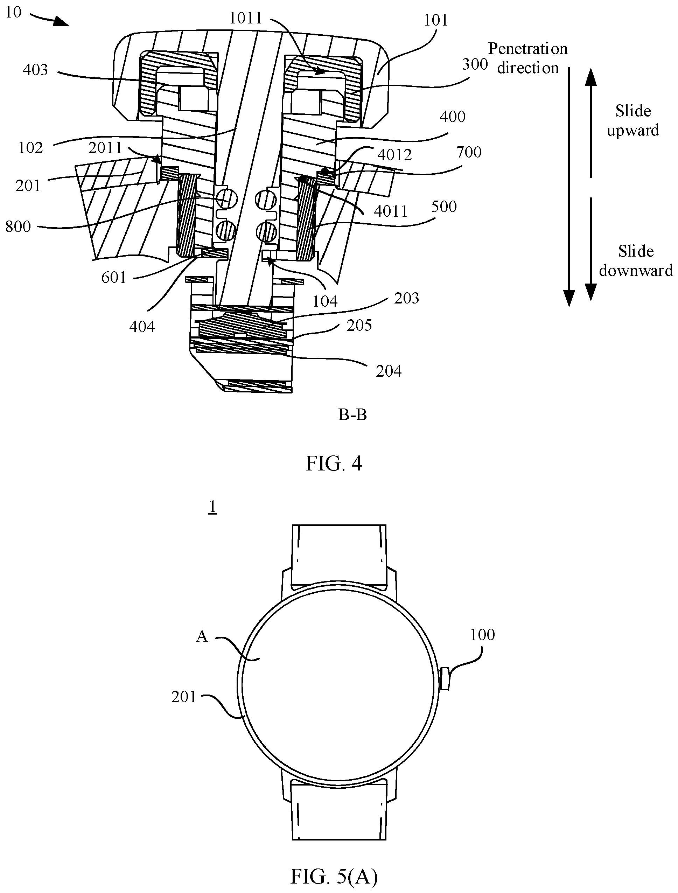

The following describes a specific structure of the foregoing control mechanism 10 with reference to FIG. 1(B) to FIG. 4. FIG. 2(A) is a top view of a partial structure in FIG. 1(A), FIG. 2(B) is a side view of FIG. 2(A), FIG. 3 is a sectional view of the operating part 100 and the fastener 200 in an S2 area of FIG. 2(A) along an A-A section, and FIG. 4 is a sectional view of the operating part 100 and the fastener 200 in an S1 area of FIG. 2(B) along a B-B section.

Specifically, as shown in FIG. 1(B) and FIG. 3, the control mechanism 10 includes the operating part 100 and the fastener 200, the operating part 100 includes a horizontal operating cap 101 and a vertical operating stem 102, and the fastener 200 includes a housing 201. A through hole 2011 is disposed in a circumferential direction of the housing 201 of the fastener 200, the operating stem 102 of the operating part 100 penetrates the through hole 2011, and the operating stem 102 may slide up and down relative to the through hole 2011 in a penetration direction in the figure. An insulation layer is coated on an outer circumferential surface that is of the operating stem 102 of the operating part 100 and that is opposite to an inner hole surface of the through hole 2011, or the insulation layer is coated on a segment of an inner circumferential surface that is of the inner hole surface of the through hole 2011 and that is opposite to the operating stem 102, and the operating part 100 is isolated from the housing 201 through insulation by using the insulation layer.

It may be understood that, in an actual design, a size of the smartwatch 1 is small, the housing 201 of the smartwatch 1 is thin (for example, a thickness of the housing 201 is generally 3 millimeters to 5 millimeters), the through hole 2011 provided on the housing 201 is small, and therefore, the operating stem 102 that penetrates the through hole 2011 is thin. Further, to prevent the operating stem 102 from breaking under an external force perpendicular to the operating stem 102, the operating part 100 further includes a crown tube 400 sleeved on the operating stem 102. The crown tube 400 may disperse the external force that directly acts on the operating stem 102, to protect the operating stem 102. In addition, the operating stem 102 may slide up and down relative to the crown tube 400 in the penetration direction in the figure. The crown tube 400 penetrates the through hole 2011 on the housing 201 and is unmovable relative to the through hole 2011. An insulation layer is coated on an outer circumferential surface that is of the crown tube 400 and that is opposite to the through hole 2011, or an insulation layer is coated on the inner hole surface that is of the through hole 2011 and that is opposite to the crown tube 400, so that the operating part 100 and the housing 201 are isolated through insulation.

In some implementations, a thickness of the insulation layer ranges from 2 microns to 5 microns. For example, the thickness range of the insulation layer may be 3 microns. In some implementations, a material of the insulation layer may be a DLC material.

In addition, in some implementations, the housing 201 may be of an annular shape or a square shape.

In some implementations, to implement a control function of the control mechanism 10 by pressing the operating cap 101, that is, to implement an ECG function by pressing the operating cap 101, a metal dome switch 203 may be disposed inside the housing 201, an end that is of the operating stem 102 and that is away from the operating cap 101 elastically abuts against a top 2031 of the metal dome switch 203, and the operating stem 102 slidably penetrates the through hole 2011 in the penetration direction.

More specifically, the fastener 200 includes an elastomer 202, a flexible printed circuit (Flexible Printed Circuit, FPC) 204, and a first bracket 205 configured to support the metal dome switch 203. An end face that is of a first end part 2021 of the elastomer 202 and that is close to the metal dome switch 203 abuts against or is in clearance fit with an end face of the top 2031 of the metal dome switch 203, and an end face that is of the first end part 2021 and that is away from the metal dome switch 203 abuts against an end face that is of the end, of the operating stem 102, away from the operating cap 101 and that is close to the metal dome switch 203, to implement the foregoing elastic abutting. In addition, a second end part 2022 of the elastomer 202 is fastened to a third portion 2053 of the first bracket 205, a bottom 2032 of the metal dome switch 203 is fastened to a surface that is of a first portion 2051 of the first bracket 205 and that faces the metal dome switch 203, a surface that is of the first portion 2051 of the first bracket 205 and that is away from the metal dome switch 203 is fastened to the FPC board 204, and a second portion 2052 of the first bracket 205 is fastened to the housing 201 (not shown in the figure).

In some implementations, to improve stability of a connection between the metal dome switch 203 and the first bracket 205, the bottom 2032 of the metal dome switch 203 is fastened to a surface that is of the first bracket 205 and that faces the bottom 2032 of the metal dome switch 203 in a glue dispensing or adhesive manner.

In some implementations, to improve stability and reliability of a connection between the first bracket 205 and the FPC board 204, the first bracket 205 and the FPC board 204 may be soldered together. Further, the first bracket 205 and the FPC board 204 may be soldered together, to prevent misplacement and falling off of the elastomer 202 when the elastomer 202 is pressed for a long time. A specific soldering manner may be spot soldering. For example, a surface that is of the first bracket 205 and that is away from the metal dome switch 203 is connected to the FPC board 204 through spot soldering by using a surface mounted technology (Surface Mounted Technology, SMT).

In some implementations, to enable the first bracket 205 to be stably fastened to the housing 201, a positioning hole may be provided on the housing 201, so that the first bracket 205 is fastened to the housing 201 by using a screw (not shown in the figure), thereby improving stability between the first bracket 205 and the housing 201.

The operating part 100 may include the operating cap 101 and the operating stem 102 extending to a watch body of the smartwatch 1 in an axial direction of the operating cap 101. The operating cap 101 is of a circular shape, and the axial direction of the operating cap 101 is a direction in which a center of the circular shape extends toward the watch body of the smartwatch 1. The operating stem 102 is of a columnar structure. In some embodiments, the control mechanism 10 further includes the crown tube 400, the crown tube 400 is sleeved on the operating stem 102, an inner surface of the crown tube 400 abuts against an outer surface of the operating stem 102, and an outer surface of the crown tube 400 abuts against the inner hole surface of the through hole 2011 on the housing 201.

The end that is of the operating stem 102 and that is away from the operating cap 101 abuts against a surface that is of the first end part 2021 of the elastomer 202 and that is away from the top 2031 of the metal dome switch 203. When being pressed downward, the operating cap 101 drives the operating stem 102 to move downward relative to the through hole 2011. When being driven by the operating cap 101 to move downward, the operating stem 102 presses the elastomer 202 that abuts against the operating stem 102, and the elastomer 202 elastically deforms downward, and generates a resilience force that pushes the operating stem 102 upward in an axial direction of the operating stem 102. At the same time, downward elastic deformation formed by the elastomer 202 presses the metal dome switch 203, so that the top 2031 of the metal dome switch 203 abuts against the bottom 2032, that is, the metal dome switch 203 is switched on. Based on this, a loop among the operating cap 101, the operating stem 102, the elastomer 202, the metal dome switch 203, the first bracket 205, and the FPC board 204 is conducted.

In some implementations, to improve electrical conductivity of the elastomer 202, a conductive coating may be added to a surface on which the elastomer 202 abuts against the operating stem 102 and a surface on which the elastomer 202 abuts against the metal dome switch 203. In addition, to prevent the conductive coating from falling off easily in a process in which the elastomer 202 is pressed and rebounds for a long time, a conductive material with a high friction coefficient may be used, to increase friction between the conductive coating and the elastomer 202, and avoid falling off of the conductive coating in a process in which the elastomer 202 is pressed and rebounds for a long time.

In addition, in some implementations, to enable an upward resilience force of the elastomer 202 to push the operating stem 102 to move in a direction away from the metal dome switch 203, and to consider a touch feeling of a user when the user presses the operating cap 101, in some embodiments, an elastic arm length of the elastomer 202 may be designed to ensure that the operating stem 102 can be rebounded by the resilience force generated by the elastomer 202. The elastic arm length of the elastomer 202 may be an empirical value or an experimental value. For example, the elastic arm length of the elastomer 202 may be 3.5 mm.

In some implementations, because an inner diameter of the crown tube 400 is greater than a diameter of the operating stem 102, to prevent the operating stem 102 from falling off from the crown tube 400 when the operating stem 102 moves in a direction away from the metal dome switch 203 under the resilience force of the elastomer 202, in some implementations, the control mechanism 10 further includes a circlip 600, an annular second slot 104 is disposed in a circumferential direction of the operating stem 102, the circlip 600 is clamped on the second slot 104, and a first end face 601 of the circlip 600 abuts against a second opening end face 404 of the crown tube 400. A size of the circlip 600 in a radial direction of the operating stem 102 is greater than the diameter of the operating stem 102, and the first end face 601 of the circlip 600 is a surface that is of the circlip 600 and that is axially perpendicular to the operating stem 102. The second opening end face 404 of the crown tube 400 is an end face away from an end of the operating cap 101. In some implementations, to ensure reliability of a connection between the circlip 600 and the operating stem 102, the circlip 600 may be fastened to the operating stem 102 in a spot soldering manner.

In some implementations, to reduce friction between the operating cap 101 and the crown tube 400 when the operating cap 101 is pressed, a first annular slot 1011 centered on the operating stem 102 is provided on the operating cap 101, and the first annular slot 1011 is configured to be clamped to the second bracket 300, so that an anti-friction effect between the operating cap 101 and the crown tube 400 is achieved, and abrasion of the operating cap 101 is reduced. In some embodiments, a material of the second bracket 300 may be an abrasion-resistant material like polyformaldehyde (polyformaldehyde, POM) or poly(ether-ether-ketone) (poly(ether-ether-ketone), PEEK).

In some implementations, to reduce friction generated when the operating stem 102 moves relative to the crown tube 400 and achieve a waterproof effect between the operating stem 102 and the crown tube 400, the control mechanism 10 includes a first waterproof part 800. One or more first slots 103 are provided on the outer surface of the operating stem 102 in an extension direction of the operating stem 102. The first slot 103 is configured to be clamped to the first waterproof part 800. When the crown 102 moves relative to the crown tube 400, the first waterproof part 800 is clamped to the first slot 103, and does not protrude to interfere with movement between the operating stem 102 and the crown tube 400, so that the waterproof effect between the operating stem 102 and the crown tube 400 can be achieved. In some implementations, a material of the first waterproof part 800 may be fluorine rubber.

In some embodiments, there may be a plurality of first slots 103, and the plurality of first slots 103 are sequentially disposed in the axial direction of the operating stem 102. Correspondingly, there are also a plurality of first waterproof parts 800, to enhance the waterproof effect between the operating stem 102 and the crown tube 400, that is, implement multi-layer waterproofing between the operating stem 102 and the crown tube 400.

In some embodiments, the first slot 103 is disposed in an area in which the crown tube 400 is sleeved on the operating stem 102, and the first waterproof part 800 is disposed between the operating stem 102 and the crown tube 400. In this way, the circlip 600 mentioned above may prevent the operating stem 102 from falling off from the crown tube 400, and may also prevent the first waterproof part 800 from falling off from the first opening end face 403 of the crown tube 400.

In some other implementations, the crown tube 400 is sleeved with a first annular structure 500, the crown tube 400 abuts against an inner hole surface of the through hole 2011 by using the first annular structure 500, and a material of the first annular structure 500 is a waterproof insulation material like rubber, so that a waterproof insulation effect between the crown tube 400 and the inner hole surface of the through hole 2011 on the housing 201 can be achieved. In addition, in some implementations, to enhance reliability of a connection between the first annular structure 500 and the crown tube 400, a third slot 402 is further provided on a surface on which the crown tube 400 abuts against the first annular structure 500. When the first annular structure 500 is sleeved on the crown tube 400, a part of the first annular structure 500 is clamped to the third slot 402, and an outer circumferential surface of the first annular structure 500 abuts against the inner hole surface of the through hole 2011.

In some implementations, the crown tube 400 further includes a flange part 401, the flange part 401 includes a first stepped surface 4011 and a second stepped surface 4012 that are opposite to each other, one end face of the first annular structure 500 abuts against the first stepped surface 4011 of the flange part 401 of the crown tube 400, and the other end face of the first annular structure 500 is flush with the second slot 104 on the operating stem 102.

In addition, to further enhance a waterproof insulation effect between the crown tube 400 and the housing 201, a second waterproof part 700 may be further disposed between the crown tube 400 and the inner hole surface of the through hole 2011 on the housing 201. The second waterproof part 700 is of an annular structure and is sleeved on the crown tube 400, and an end face of the second waterproof part 700 abuts against the second stepped surface 4012.

When the user needs to use an electrocardiogram (electrocardiogram, ECG) function, a finger of a right hand (or a finger of a left hand) of the user presses the operating cap 101, so that the operating stem 102 moves relative to the housing 201 toward a direction of the metal dome switch 203, and presses the elastomer 202, so that the elastomer 202 elastically deforms and generates an upward resilience force. At the same time, the metal dome switch 203 is switched on, a circuit between the metal dome switch 203 and the FPC board 204 is conducted, and a loop is formed among the finger of the right hand (or the finger of the left hand) of the user, the operating cap 101, the operating stem 102, the elastomer 202, the metal dome switch 203, and the FPC board 204.

After the user releases the finger, the operating stem 102 moves relative to the housing 201 under the resilience force of the elastomer 202 in the direction away from the metal dome switch 203, and the metal dome switch 203 is switched off, so that the circuit between the metal dome switch 203 and the FPC board 204 is disconnected, and further, the loop among the finger of the right hand (or the finger of the left hand) of the user, the operating cap 101, the operating stem 102, the elastomer 202, the metal dome switch 203, and the FPC board 204 is disconnected.

Based on the foregoing structure, when the user wears the smartwatch (for example, the user wears the smartwatch on the left hand) and uses the ECG function, between the left hand of the user wearing the smartwatch and the finger of the right hand of the user pressing the operating cap, a loop is formed by using the operating cap, the operating stem, the elastomer, the FPC board, and a rear housing that is of the smartwatch and that is in contact with the left hand of the user, to provide a basis for implementing the ECG function.

In the present case, a manner of implementing the foregoing elastic abutting and a conductive path in a control mechanism 10' for implementing the ECG function in the smartwatch 1 are also improved.

Specifically, in the smartwatch 1 the conductive path in the control mechanism 10' includes an ECG electrode 1, an ECG electrode 2, and a rear housing. In some embodiments, the ECG electrode 1 and the ECG electrode 2 may be dry electrodes.

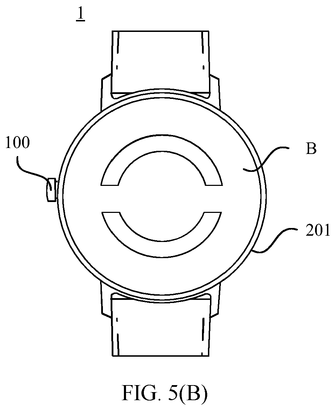

Specifically, as shown in FIG. 5(A) and FIG. 5(B), FIG. 5(A) shows a front surface A of the smartwatch 1, and FIG. 5(B) shows a rear surface B of the smartwatch 1. A front housing of the smartwatch 1 covers the front surface A, a rear housing of the smartwatch 1 covers the rear surface B, and an annular structure between the rear housing and the front housing is the housing 201 of the smartwatch 1.

In embodiments the ECG electrode 1 may be disposed on the operating part 100, and the ECG electrode 2 may be disposed on the rear housing.

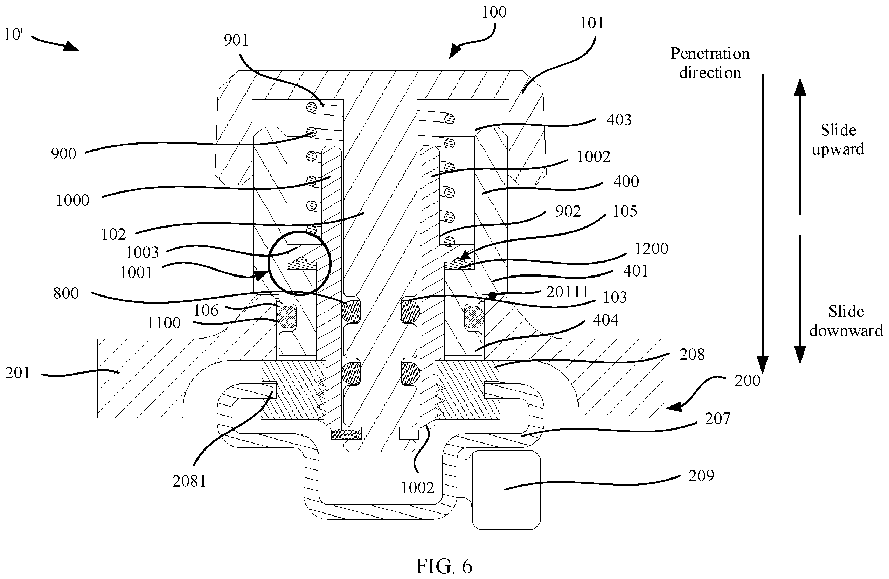

The following describes the control mechanism 10' of the smartwatch 1 in this embodiment with reference to FIG. 6. As described above, a main difference between the control mechanism 10' in FIG. 6 and the control mechanism of the smartwatch 1 in FIG. 3 and FIG. 4 lies in that conductive paths in the two control mechanisms are different and manners of implementing the foregoing elastic abutting are different. When the control mechanism in FIG. 3 and FIG. 4 implements the ECG function, the conductive path in the control mechanism is, as described above, a loop formed among the finger of the right hand (or the finger of the left hand) of the user, the operating cap 101, the operating stem 102, the elastomer 202, the metal dome switch 203, and the FPC board 204, and the conductive path in FIG. 6 is simpler than the conductive path in FIG. 3 and FIG. 4. In addition, in FIG. 3 and FIG. 4, elastic abutting between the operating stem 102 and the top 2031 of the metal dome switch 203 is implemented by using the elastomer 202 disposed between the operating stem 102 and the metal dome switch 203, and in FIG. 6, elastic abutting between the operating stem 102 and the top 2031 of the metal dome switch 203 is implemented by using a spring sleeved on the operating stem 102. Details are described below.

As shown in FIG. 6, the control mechanism 10' includes an operating part 100 and a fastener 200. The operating part 100 includes a horizontal operating cap 101, a vertical operating stem 102, and a crown tube 400 sleeved on the operating stem 102, the operating stem 102 is slidable relative to the crown tube 400 in a penetration direction, the crown tube 400 penetrates a through hole 2011, the crown tube 400 is unmovable relative to the through hole 2011, and an insulation layer is coated on a contact surface on which the crown tube 400 abuts against an outer edge 20111 of the through hole 2011 in the penetration direction and/or on a surface that is of the outer edge 20111 of the through hole 2011 and that is opposite to the contact surface, so that the operating part 100 is isolated from the fastener 200 through insulation. The outer edge 20111 of the through hole 2011 is a part that is of the through hole 2011 and that abuts against a stepped surface of an outer surface of a flange part 401 of the crown tube 400 in the penetration direction.

More specifically, the operating part 100 further includes a sleeve structure 1000 disposed between the crown tube 400 and the operating stem 102. The sleeve structure 1000 includes a body part 1002 and an extension part 1003, a part that is of the extension part 1003 and that is connected to the body part 1002 forms a flange part 1001 of the sleeve structure 1000, the body part 1003 penetrates the crown tube 400, the extension part 1003 abuts against the crown tube 400 in the penetration direction, and the operating stem 102 may slide up and down relative to the sleeve structure 1000 in the penetration direction. The extension part 1003 is perpendicular to the penetration direction. The fastener 200 includes a housing 201, the through hole 2011 is provided on an outer circumferential surface of the housing 201, and a metal dome switch 203 and a first elastic structure 900 are disposed inside the housing 201. The crown tube 400, and the body part 1002 and the extension part 1003 of the sleeve structure 1000 enclose an accommodating cavity, the accommodating cavity is configured to accommodate the first elastic structure 900, a first end 901 of the first elastic structure 900 in the penetration direction abuts against the extension portion 1002 of the sleeve structure 1000, and a second end 902 of the first elastic structure 900 is disposed opposite to the operating cap 101. An end that is of the operating stem 102 and that is away from the operating cap 101 abuts against the metal dome switch 203, so that an end of the operating stem 102 elastically abuts against a top 2031 of the metal dome switch 203, and further, the metal dome switch 203 is switched on/off by pressing/releasing the operating cap 101, thereby implementing a control function of the control mechanism.

In some implementations, the first end 901 of the first elastic structure 900 is higher than a first opening end face 403 of the crown tube 400 by a preset distance, so that the first elastic structure 900 can elastically deform downward under pressing, and generate an upward resilience force.

In some implementations, the first elastic structure 900 may be a spring, and the spring is sleeved on the body part 1002 of the sleeve structure 1000.

In some embodiments, the control mechanism 10' further includes the sleeve structure 1000. The sleeve structure 1000 is sleeved on the operating stem 102, and an inner hole surface of the sleeve structure 1000 abuts against a circumferential surface of the operating stem 102. In some embodiments, to enhance a waterproof effect between the operating stem 102 and the inner hole surface of the sleeve structure 1000, one or more first slots 103 may be sequentially disposed on the operating stem 102 in an axial direction, and are configured to be clamped to one or more first waterproof parts 800.

In some implementations, to enhance an insulation effect between the crown tube 400 and the sleeve structure 1000, a first insulation part 1200 is disposed between the flange part 401 of the crown tube 400 and the flange part 1001 of the sleeve structure 1000. In some embodiments, the first insulation part 1200 is of an annular structure. In some embodiments, to improve reliability of a connection between the first insulation part 1200 and the sleeve structure 1000, a fourth slot 105 may be disposed on a surface on which the flange part 1001 of the sleeve structure 1000 abuts against the first insulation part 1200. When the flange part 1001 of the sleeve structure 1000 is clamped to the flange part 401 of the crown tube 400, a part of the first insulation part 1200 is pressed into the fourth slot 105, to implement interference fit between the first insulation part 1200 and the sleeve structure 1000.

In some embodiments, to achieve a waterproof insulation effect between the crown tube 400 and the housing 201, a fifth slot 106 may be disposed on a surface on which the flange part 401 of the crown tube 400 is in contact with the housing 201, and configured to be clamped to a third waterproof part 1100. In some embodiments, the third waterproof part 1100 is of an annular structure.

In some implementations, a third bracket 207, a first fastening structure 208, and a second fastening structure 209 are further disposed inside the housing 201 of the fastener 200. The first fastening structure 208 is of an annular structure, and is sleeved at an end that is of the sleeve structure 1000 and that is away from the operating cap 101. In some embodiments, to enhance stability of a connection between the first fastening structure 208 and the sleeve structure 1000, a thread may be disposed on an inner wall of the first fastening structure 208, and a thread is also disposed on a part that is of the sleeve structure 1000 and that extends out of the crown tube 400, so that the first fastening structure 208 is threadedly connected to the sleeve structure 1000.

A sixth slot 2081 is disposed on an outer side of the first fastening structure 208, and is configured to be clamped to the third bracket 207. The third bracket 207 is fastened to the second fastening structure 209, and the second fastening structure 209 is fastened to an FPC board (not shown in the figure).

Based on the foregoing structure, when the user needs to use an electrocardiogram (electrocardiogram, ECG) function, the finger of the right hand (or the finger of the left hand) of the user presses the operating cap 101, and a loop is formed among the finger of the right hand (or the finger of the left hand) of the user, the operating cap 101, the operating stem 102, the first elastic structure 900, the sleeve structure 1000, the first fastening structure 208, the third bracket 207, the second fastening structure 209, a metal dome switch (not shown in the figure), and the FPC board, and the FPC board converts a detected heart rate signal of the user into an electrical signal, and transmits the electrical signal to a detection circuit (not shown in the figure), to implement the ECG function.

After the user releases the finger, the operating cap 101 moves relative to the housing 201 in a direction away from the first fastening structure 208 under a resilience force of the first elastic structure 900, and at the same time, a loop formed among the finger of the right hand (or the finger of the left hand) of the user, the operating cap 101, the operating stem 102, the first elastic structure 900, the sleeve structure 1000, the first fastening structure 208, the third bracket 207, the second fastening structure 209, the metal dome switch (not shown in the figure), and the FPC board is disconnected.

It can be learned that the conductive path shown in FIG. 6 is simpler than the conductive paths in FIG. 3 and FIG. 4. This can further reduce complexity of the structure of the smartwatch 1 to some extent, and reduce manufacturing costs of the smartwatch 1.

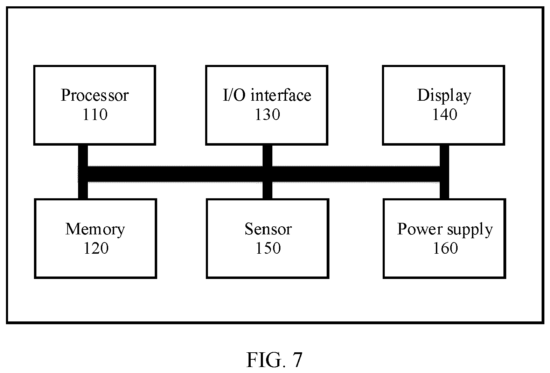

The following describes a diagram of a structure of the smartwatch 1 in an embodiment of the present invention. FIG. 7 is a diagram of a structure of a smartwatch 1 according to some embodiments. As shown in FIG. 7, the smartwatch 1 includes a processor 110, a memory 120, an input/output interface 130, a display 140, a sensor 150, and a power supply 160.

The processor 110 may be directly or indirectly connected to another module of the smartwatch 1. For example, the memory 120, the input/output interface 130, the display 140, the sensor 150, and the power supply 160 may all be connected to the processor 110 through a bus 170. The processor 110 may include one or more processing units.

The module on the smartwatch 1 may be controlled by a plurality of processing units. For example, the sensor 150 on the smartwatch 1 may be controlled by a first processing unit, and the display 140 on the smartwatch 1 may be controlled by a second processor. The first processor and the second processor may communicate with each other.

The memory 120 may be configured to store computer-executable program code. The executable program code includes instructions. The processor 110 runs the instructions of the memory 120, to implement various functions and data processing of the smartwatch 1. The memory 120 may include a program storage area and a data storage area. The program storage area may store an application required by at least one function (for example, a heart rate detection function). The data storage area may store data (for example, heart rate data of the user collected by the ECG) created during use of the smartwatch 1, and the like. In addition, the memory 120 may include a high-speed random access memory, and may further include a non-volatile memory, for example, at least one magnetic disk storage device, a flash memory device, or a universal flash storage (universal flash storage, UFS).

The input/output interface 130 may receive data from an input/output module of the smartwatch 1 or from another smartwatch 1. The input/output module may include, for example, a touch panel, a mechanical button or a virtual button, a camera, a microphone, or a speaker.

The display 140 is configured to display an image, a video, and the like. The display 140 includes a display panel. The display panel may be a liquid crystal display (liquid crystal display, LCD), an organic light-emitting diode (organic light-emitting diode, OLED), an active-matrix organic light-emitting diode (active-matrix organic light emitting diode, AMOLED), a flexible light-emitting diode (flexible light-emitting diode, FLED), a mini-LED, a micro-LED, a micro-OLED, a quantum dot light-emitting diode (quantum dot light emitting diode, QLED), or the like.

The smartwatch 1 further includes the sensor 150. The sensor 150 may be configured to sense one or more types of parameters, for example, a pressure parameter, a light parameter, a touch parameter, a heat parameter, a motion parameter, a biological parameter, or the like. For example, the sensor 150 may include a thermal sensor, a position sensor, an optical proximity sensor, an accelerometer, a pressure sensor, a gyroscope, a magnetometer, and the like. The sensor 150 may further include an ECG device described in embodiments of the present invention. A motor of the ECG device may be exposed to a housing of the smartwatch 1100.

The power supply 160 may be configured to supply power to the smartwatch 1. The power supply 160 may be one or more batteries. In addition, the power supply 160 may alternatively be a power connector or a power cable. The power connector or the power cable is configured to connect the smartwatch 1 to another power supply, for example, a socket, for charging.

In some embodiments, the smartwatch 1 further includes an antenna and a communication module, so that the smartwatch 1 can communicate with a network and another smartwatch 1 by using a wireless communication technology. The wireless communication includes a wireless fidelity (wireless fidelity, Wi-Fi) network, Bluetooth, an Ethernet connection, and the like.

## Claims

1. A control mechanism (10), wherein the control mechanism (10) comprises:
a fastener (200) and an operating part (100) isolated from the fastener (200) through insulation, wherein
the fastener (200) is provided with a through hole (2011), the operating part (100) penetrates the through hole (2011), and an insulation layer is coated on at least a segment of an inner circumferential surface that is of the through hole (2011) and that is opposite to the operating part (100) and/or on an outer circumferential surface that is of the operating part (100) and that is opposite to the segment of the inner circumferential surface; and/or
an insulation layer is coated on a contact surface on which the operating part (100) abuts against an outer edge of the through hole (2011) in a penetration direction and/or on a surface that is of the outer edge of the through hole (2011) and that is opposite to the contact surface; and
the fastener (200) and the operating part (100) are isolated through insulation by using the insulation layer;
wherein the operating part (100) is shaped like T, the operating part (100) comprises a horizontal operating cap (101) and a vertical operating stem (102), the operating stem (102) penetrates the through hole (2011) and slides relative to the through hole (2011) in the penetration direction, and the outer circumferential surface is an outer circumferential surface of the operating stem (102);
wherein the fastener (200) comprises a housing (201), the through hole (2011) is provided on an outer circumferential surface of the housing (201), and a metal dome switch (203) is disposed inside the housing (201); and the operating stem (102) slidably penetrates the through hole (2011) in the penetration direction, and the metal dome switch (203) is switched on/off by pressing/releasing the operating cap (101);
**characterised in that** an end that is of the operating stem and that is away from the operating cap elastically abuts against a top of the metal dome switch, and **in that** an elastomer (202) and a first bracket (205) supporting the metal dome switch (203) are further disposed inside the housing (201), one end of the elastomer (202) is fastened to the first bracket (205), the other end of the elastomer (202) abuts against the top (2031) of the metal dome switch (203), and the end that is of the operating stem (102) and that is away from the operating cap (101) elastically abuts against the metal dome switch (203) through the other end of the elastomer (202).

2. The control mechanism (10) according to claim 1, wherein the operating part (100) is shaped like T, the operating part (100) comprises a horizontal operating cap (101), a vertical operating stem (102), and a crown tube (400) sleeved on the operating stem (102), the operating stem (102) is slidable relative to the crown tube (400) in the penetration direction, the crown tube (400) penetrates the through hole (2011), the crown tube (400) is unmovable relative to the through hole (2011), and the outer circumferential surface is an outer circumferential surface of the crown tube (400).

3. The control mechanism (10) according to claim 2, wherein the contact surface is a surface on which the crown tube (400) abuts against the outer edge of the through hole (2011) in the penetration direction.

4. The control mechanism (10) according to any one of claims 1 to 2, wherein a thickness of the insulation layer ranges from 2 microns to 5 microns.

5. The control mechanism (10) according to any one of claims 1 to 2, wherein the insulation layer comprises diamond-like carbon.

6. A wearable device, comprising the control mechanism (10) according to any one of claims 1 to 5 and a display, wherein the display is disposed on a fastener (200) of the control mechanism (10).

## Patentansprüche

1. Steuerungsmechanismus (10), wobei der Steuerungsmechanismus (10) Folgendes umfasst:
ein Befestigungselement (200) und ein Betätigungsteil (100), das über eine Isolation von dem Befestigungselement (200) getrennt ist, wobei
das Befestigungselement (200) mit einem Durchgangsloch (2011) versehen ist, das Betätigungsteil (100) das Durchgangsloch (2011) durchdringt und eine Isolationsschicht auf mindestens einem Segment einer inneren Umfangsoberfläche, die dem Durchgangsloch (2011) angehört und die dem Betätigungsteil (100) gegenüberliegt, und/oder auf einer äußeren Umfangsoberfläche, die dem Betätigungsteil (100) angehört und die dem Segment der inneren Umfangsoberfläche gegenüberliegt, geschichtet ist; und/oder
eine Isolationsschicht auf einer Kontaktoberfläche, auf der das Betätigungsteil (100) an einem äußeren Rand des Durchgangslochs (2011) in einer Durchdringungsrichtung anliegt, und/oder auf einer Oberfläche, die dem äußeren Rand des Durchgangslochs (2011) angehört und die der Kontaktoberfläche gegenüberliegt, geschichtet ist; und
das Befestigungselement (200) und das Betätigungsteil (100) unter Verwendung der Isolationsschicht über die Isolation getrennt sind;
wobei das Betätigungsteil (100) wie ein T geformt ist, das Betätigungsteil (100) eine horizontale Betätigungskappe (101) und einen vertikalen Betätigungsstiel (102) umfasst, der Betätigungsstiel (102) das Durchgangsloch (2011) durchdringt und in Bezug auf das Durchgangsloch (2011) in der Durchdringungsrichtung gleitet und die äußere Umfangsoberfläche eine äußere Umfangsoberfläche des Betätigungsstiels (102) ist;
wobei das Befestigungselement (200) ein Gehäuse (201) umfasst, das Durchgangsloch (2011) auf einer äußeren Umfangsoberfläche des Gehäuses (201) bereitgestellt ist und ein Metallkuppelschalter (203) innerhalb des Gehäuses (201) angeordnet ist; und
der Betätigungsstiel (102) das Durchgangsloch (2011) in der Durchdringungsrichtung gleitbar durchdringt und der Metallkuppelschalter (203) durch Drücken/Loslassen der Betätigungskappe (101) ein-/ausgeschaltet wird;
**dadurch gekennzeichnet, dass** ein Ende, das dem Betätigungsstiel angehört und das von der Betätigungskappe abgewandt ist, elastisch an einer Oberseite des Metallkuppelschalters anliegt, und dass
ein Elastomer (202) und eine erste Halterung (205), die den Metallkuppelschalter (203) stützt, ferner innerhalb des Gehäuses (201) angeordnet sind, ein Ende des Elastomers (202) an der ersten Halterung (205) befestigt ist, das andere Ende des Elastomers (202) an der Oberseite (2031) des Metallkuppelschalters (203) anliegt und das Ende, das dem Betätigungsstiel (102) angehört und das von der Betätigungskappe (101) abgewandt ist, über das andere Ende des Elastomers (202) elastisch an dem Metallkuppelschalter (203) anliegt.

2. Steuerungsmechanismus (10) nach Anspruch 1, wobei das Betätigungsteil (100) wie ein T geformt ist, das Betätigungsteil (100) eine horizontale Betätigungskappe (101), einen vertikalen Betätigungsstiel (102) und eine Kronentube (400), die auf dem Betätigungsstiel (102) aufgesetzt ist, umfasst, der Betätigungsstiel (102) in Bezug auf die Kronentube (400) in der Durchdringungsrichtung gleitbar ist, die Kronentube (400) das Durchgangsloch (2011) durchdringt, die Kronentube (400) in Bezug auf das Durchgangsloch (2011) unbewegbar ist und die äußere Umfangsoberfläche eine äußere Umfangsoberfläche der Kronentube (400) ist.

3. Steuerungsmechanismus (10) nach Anspruch 2, wobei die Kontaktoberfläche eine Oberfläche ist, auf der die Kronentube (400) in der Durchdringungsrichtung an dem äußeren Rand des Durchgangslochs (2011) anliegt.

4. Steuerungsmechanismus (10) nach einem der Ansprüche 1 bis 2, wobei eine Dicke der Isolationsschicht von 2 Mikron bis 5 Mikron reicht.

5. Steuerungsmechanismus (10) nach einem der Ansprüche 1 bis 2, wobei die Isolationsschicht einen diamantartigen Kohlenstoff umfasst.

6. Wearable-Vorrichtung, umfassend den Steuerungsmechanismus (10) nach einem der Ansprüche 1 bis 5 und eine Anzeige, wobei die Anzeige auf einem Befestigungselement (200) des Steuerungsmechanismus (10) angeordnet ist.

## Revendications

1. Mécanisme de commande (10), dans lequel le mécanisme de commande (10) comprend :
un élément de fixation (200) et une partie d'actionnement (100) isolée de l'élément de fixation (200) par une isolation, dans lequel
l'élément de fixation (200) est muni d'un trou traversant (2011), la partie d'actionnement (100) pénètre dans le trou traversant (2011), et une couche isolante est appliquée sur au moins un segment d'une surface circonférentielle intérieure qui est du trou traversant (2011) et qui est opposée à la partie d'actionnement (100) et/ou sur une surface circonférentielle extérieure qui est de la partie d'actionnement (100) et qui est opposée au segment de la surface circonférentielle intérieure ; et/ou
une couche isolante est appliquée sur une surface de contact sur laquelle la partie d'actionnement (100) vient en butée contre un bord extérieur du trou traversant (2011) dans une direction de pénétration et/ou sur une surface qui est du bord extérieur du trou traversant (2011) et qui est opposée à la surface de contact ; et
l'élément de fixation (200) et la partie d'actionnement (100) sont isolées par une isolation à l'aide de la couche isolante ;
dans lequel la partie d'actionnement (100) est en forme de T, la partie d'actionnement (100) comprend un capuchon d'actionnement horizontal (101) et une tige d'actionnement (102) verticale, la tige d'actionnement (102) pénètre dans le trou traversant (2011) et glisse par rapport au trou traversant (2011) dans la direction de pénétration, et la surface circonférentielle extérieure est une surface circonférentielle extérieure de la tige d'actionnement (102) ;
dans lequel l'élément de fixation (200) comprend un boîtier (201), le trou traversant (2011) est prévu sur une surface circonférentielle extérieure du boîtier (201), et un commutateur de dôme métallique (203) est disposé à l'intérieur du boîtier (201) ; et
la tige d'actionnement (102) pénètre de manière coulissante dans le trou traversant (2011) dans la direction de pénétration, et le commutateur de dôme métallique (203) est allumé/éteint en appuyant/relâchant le capuchon d'actionnement (101) ;
**caractérisé en ce qu'**une extrémité qui est de la tige d'actionnement et qui est éloignée du capuchon d'actionnement vient en butée de manière élastique contre une portion supérieure du commutateur de dôme métallique, et **en ce que**
un élastomère (202) et un premier support (205) supportant le commutateur de dôme métallique (203) sont en outre disposés à l'intérieur du boîtier (201), une extrémité de l'élastomère (202) est fixée au premier support (205), l'autre extrémité de l'élastomère (202) vient en butée contre la portion supérieure (2031) du commutateur de dôme métallique (203), et l'extrémité qui est de la tige d'actionnement (102) et qui est éloignée du capuchon d'actionnement (101) vient en butée de manière élastique contre le commutateur de dôme métallique (203) par l'autre extrémité de l'élastomère (202).

2. Mécanisme de commande (10) selon la revendication 1, dans lequel la partie d'actionnement (100) est en forme de T, la partie d'actionnement (100) comprend un capuchon d'actionnement horizontal (101), une tige d'actionnement (102) verticale et un tube de couronne (400) gainé sur la tige d'actionnement (102), la tige d'actionnement (102) est coulissante par rapport au tube de couronne (400) dans la direction de pénétration, le tube de couronne (400) pénètre dans le trou traversant (2011), le tube de couronne (400) est immobile par rapport au trou traversant (2011), et la surface circonférentielle extérieure est une surface circonférentielle extérieure du tube de couronne (400).

3. Mécanisme de commande (10) selon la revendication 2, dans lequel la surface de contact est une surface sur laquelle le tube de couronne (400) vient en butée contre le bord extérieur du trou traversant (2011) dans la direction de pénétration.

4. Mécanisme de commande (10) selon l'une quelconque des revendications 1 et 2, dans lequel une épaisseur de la couche isolante varie de 2 microns à 5 microns.

5. Mécanisme de commande (10) selon l'une quelconque des revendications 1 et 2, dans lequel la couche isolante comprend du carbone de type diamant.

6. Dispositif pouvant être porté sur soi, comprenant le mécanisme de commande (10) selon l'une quelconque des revendications 1 à 5 et un affichage, dans lequel l'affichage est disposé sur un élément de fixation (200) du mécanisme de commande (10).
